(19) Europäisches Patentamt
European Patent Office
Office européen des brevets

(11) EP 2 740 771 B1

(12) EUROPEAN PATENT SPECIFICATION

(45) Date of publication and mention
of the grant of the patent:
19.07.2023 Bulletin 2023/29

(21) Application number: 12819908.0

(22) Date of filing: 01.08.2012

(51) International Patent Classification (IPC):
C09C 3/06 (2006.01)     A61K 8/19 (2006.01)
A61K 8/27 (2006.01)     A61K 8/29 (2006.01)
C09C 1/00 (2006.01)     C09C 3/08 (2006.01)
A61Q 17/04 (2006.01)     A61K 8/02 (2006.01)
A61K 8/04 (2006.01)     B82Y 30/00 (2011.01)
C09C 1/36 (2006.01)     C09C 1/04 (2006.01)
C09C 1/24 (2006.01)     C09C 1/02 (2006.01)
C09C 1/30 (2006.01)     B05D 5/00 (2006.01)
C09C 1/40 (2006.01)     C09C 3/00 (2006.01)
C09C 3/12 (2006.01)     G02B 5/20 (2006.01)

(52) Cooperative Patent Classification (CPC):
C09C 1/3661; A61K 8/0266; A61K 8/04;
A61K 8/19; A61K 8/27; A61K 8/29; A61Q 17/04;
B82Y 30/00; C09C 1/02; C09C 1/024; C09C 1/043;
C09C 1/24; C09C 1/3054; C09C 1/3063;
C09C 1/3081;                        (Cont.)

(86) International application number:
PCT/JP2012/069583

(87) International publication number:
WO 2013/018828 (07.02.2013 Gazette 2013/06)

(54) **COMPOSITE POWDER AND METHOD FOR PRODUCING SAME**

VERBUNDSTOFFPULVER UND HERSTELLUNGSVERFAHREN DAFÜR

POUDRE COMPOSITE, ET PROCÉDÉ DE FABRICATION DE CELLE-CI

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 03.08.2011 JP 2011169747

(43) Date of publication of application:
11.06.2014 Bulletin 2014/24

(73) Proprietor: Sakai Chemical Industry Co., Ltd.
Sakai-shi, Osaka 590-8502 (JP)

(72) Inventors:
• ASHIDA, Takuro
Osaka 590-0985 (JP)
• GOUDA, Nanae
Iwaki-shi
Fukushima 971-8183 (JP)

(74) Representative: Isarpatent
Patent- und Rechtsanwälte Barth
Charles Hassa Peckmann & Partner mbB
Friedrichstrasse 31
80801 München (DE)

(56) References cited:
JP-A- 2 247 109     JP-A- 6 299 087
JP-A- 56 020 067     JP-A- H04 506 674
JP-A- H06 502 661     JP-A- 2005 290 137
JP-A- 2006 052 299     JP-A- 2007 051 188
JP-B- 31 004 535     US-A1- 2006 034 879
US-A1- 2008 031 832     US-A1- 2009 260 297

**EP 2 740 771 B1**

(52) Cooperative Patent Classification (CPC): (Cont.)
**C09C 1/309; C09C 1/3669; C09C 1/3684;
C09C 1/3692; C09C 1/407; C09C 3/006;
C09C 3/063;** A61K 2800/412; A61K 2800/623;
A61K 2800/63; A61K 2800/651; C01P 2002/85;
C01P 2004/04; C01P 2004/51; C01P 2004/62;
C01P 2004/64; C09C 3/08; C09C 3/12

2

**Description**

TECHNICAL FIELD

The present invention relates to a composite powder and a method for producing the same.

BACKGROUND OF THE DISCLOSURE

Inorganic powders have been widely used as fillers, colorants, or the like. Recently, materials having new functions such as ultraviolet blocking performance, heat ray blocking performance, and a catalyst function have been developed by techniques for controlling particles.

However, when an inorganic powder is mixed in plastics, inks, or cosmetics, some problems may occur, for example, the inorganic powder itself may react with other components, or the chemical bond of other components may be broken by the catalyst activity of the inorganic powder. In particular, titanium oxide and zinc oxide are known to promote the aging of the resin by the catalyst activity of their surfaces. It is known that, in zinc oxide or barium carbonate, each metal ion is eluted in the presence of water.

In order to solve the problems, Patent Document 1 and Patent Document 2 propose zinc oxide subjected to a surface treatment with silica. In this method, since the contact between water and zinc oxide is prevented only at the hydrophilic silica layer, the elution of ions can be prevented in a short period. However, it is found that when the elution of ions is evaluated from a long-term perspective, the elution of Zn is not prevented completely. Further, in order to enhance inactivation, the amount of treated silica should be increased. As a result, since the content of the inorganic powder having the function is decreased, for example, the zinc oxide microparticles have a disadvantage that the ultraviolet blocking performance thereof is deteriorated. Further, suppression of the surface activity of titanium oxide by treating titanium oxide with silica is proposed, as described in Patent Document 3. The method is essentially a method in which zinc oxide is treated with silica. For inactivating zinc titanium oxide, the amount of silica should be increased.

Patent Document 4 proposes that dispersibility in water and stability during storage of a metal oxide are improved, and the elution of metal ions and surface activity are suppressed by covering the metal oxide with silica and a certain polymer. However, the certain polymer is an acrylic resin such as polymethyl methacrylate, and if any organic solvent that dissolves the polymer, for example, an aromatic hydrocarbon, an ester, or a ketone, is present with the polymer, the solvent may dissolve the layer of the polymer. Further, since three steps of wet surface treatment is required, it is expected that the cost is increased, and the resin has poor thermal stability.

Patent Document 5 describes zinc oxide particles whose surface is covered with silicon oxide, and further describes that the particles are treated with organopolysiloxane. However, by covering the surface of the particles with silicon oxide alone, the elution of Zn cannot be prevented completely from a long-term perspective although the elution of ions can be prevented in a short period. Further, when the outermost surface of the particles is subjected to a water-repellent treatment, the particles have a problem that the particles are hardly dispersed in an aqueous medium, and hardly usable in an aqueous medium.

Patent Document 6 describes spherical silica particles in which titanium oxide or zinc oxide is dispersed. However, when the above-mentioned silica is contained in a sunscreen, since titanium oxide or zinc oxide having ultraviolet blocking performance is present in silica, there is a problem that gaps tend to be formed among silica particles and that the sunscreen cannot block the ultraviolet light uniformly from the skin. Further, since the amount of silica is increased, there is a disadvantage that the ultraviolet cut-off efficiency is deteriorated.

US 2006/034879 A1 relates to scale-like composite particles having oily feel as well as soft and smooth feel, wherein the scale-like composite particles include inorganic oxide particles having an average diameter in a range from 0.1 to 3.0 um and adhered in a single layer to a surface of resin-coated scale-like inorganic particles.

JP 2 247109A discloses a cosmetic, obtained by sufficiently dispersing titanium oxide having 30-70 nm average particle diameter in water so as to provide 5-40wt.% content thereof, adding an aqueous solution of a water-soluble alkali silicate therein so as to afford 1-4wt.% content thereof based on the titanium oxide expressed in terms of SiO2, sufficiently stirring the mixture, then adding an aqueous solution of a water-soluble Al compound thereto so as to provide 6-12wt.% content thereof based on the titanium oxide expressed in terms of Al2O3, neutralizing the resultant mixture, coating the titanium oxide with the hydrated mixture, adding a silicone oil thereto while sufficiently stirring the obtained mixture, heating and calcining the mixture and providing treated powder and containing 1-25wt.% resultant powder.

US 2008/031832 A1 provides a particle of TiO2 or ZnO which has been doped with one or more other elements such that the concentration of dopant in the surface of the particle is greater than that at the core of the particle, and compositions containing such particles for use as sunscreens or in veterinary, agricultural or horticultural compositions or as coatings for plastics and other materials.

PRIOR TECHNICAL DOCUMENTS

PATENT DOCUMENTS

[Patent Document 1] Japanese Kokai Publication 2007-16111
[Patent Document 2] Japanese Kokai Publication Hei 3-183620
[Patent Document 3] Japanese Kokai Publication Hei 11-217219
[Patent Document 4] Japanese Kokai Publication 2008-266283
[Patent Document 5] Japanese Kokai Publication Hei 11-302015
[Patent Document 6] Japanese Kokai Publication Hei 06-127932

SUMMARY OF INVENTION

PROBLEM TO BE SOLVED BY THE INVENTION

An object of the present invention is to provide a composite powder that is stable even if it is present in water for a long time. The composite powder acquires hydrophilicity by treating the outermost layer thereof with a Si compound and/or an Al compound. Water is barriered by the inner water-repellent organic compound, and the elution of ions is prevented by avoiding the direct contact between the inorganic powder and water. Further, the composite powder suffers from no change in the pH in water for a long time. Further, the composite powder is effective for the inactivation of the powder.

MEANS FOR SOLVING OBJECT

The present invention relates to a composite powder, comprising:

an inorganic powder;
a first coating layer containing a water-repellent organic compound on the surface of the inorganic powder; and
a second coating layer which contains at least one compound selected from the group consisting of silicon oxide, silicon oxide hydrate, aluminum oxide, and aluminum hydroxide, and which is formed on the first coating layer, wherein said inorganic powder is titanium oxide, zinc oxide, or cerium oxide, and the average particle diameter thereof is from 10 to 200 nm, and said water-repellent organic compound is a silicone oil.

Preferably, the composite powder has a pH of 6 to 8 when the composite powder is dispersed in water and allowed to stand at 60°C for 2 weeks.
Described but not according to the invention is that the water-repellent organic compound is at least one selected from the group consisting of an alkylsilane, an alkyl titanate, an alkyl aluminate, a metallic soap, an amino acid, and an amino acid salt.
The inorganic powder is preferably an inorganic powder having ultraviolet blocking effect.
The inorganic powder is zinc oxide, titanium oxide, or cerium oxide.
The present invention also relates to a method for producing any one of the composite powders as described above, including the steps of: (1) dispersing an inorganic powder subjected to a surface treatment with a water-repellent organic compound in a dispersion medium; and (2) subjecting the inorganic powder to a surface treatment with a Si source compound and/or an Al source compound.
The present invention also relates to a method for producing any one of the composite powders as described above, including the steps of: (1) dispersing an inorganic powder subjected to a surface treatment with a water-repellent organic compound in water; and (2) subjecting the inorganic powder to a surface treatment with a Si source compound and/or an Al source compound wherein said inorganic powder is titanium oxide, zinc oxide, or cerium oxide, and the average particle diameter thereof is from 10 to 200 nm, and said water-repellent organic compound is a silicone oil.
The present invention also provides a cosmetic, containing the composite powder as described above.

EFFECT OF THE INVENTION

The present inventors have found, as a result of intensive development and research in order to solve the problems, that the surface activity of the inorganic powder and the elution of ions can be suppressed remarkably by forming a first coating layer containing a water-repellent organic compound, and forming a second coating layer containing at least one compound selected from the group consisting of silicon oxide, silicon oxide hydrate, aluminum oxide, and aluminum hydroxide, on the surface of an inorganic powder. According to the finding, the present invention was accomplished.

The composite powder obtained by the present invention can be dispersed in an aqueous medium while remarkably suppressing the surface activity of the inorganic powder. Therefore, the composite powder can be stably dispersed also in aqueous resin compositions, coating compositions, or cosmetics. Further, since the elution of ions is suppressed, the composite powder can be formed into a formulation having good stability during storage. In addition, a water-repellent powder having remarkably suppressed surface activity can be obtained by subjecting the outermost layer to a surface treatment with silicone, for example.

BRIEF DESCRIPTION OF THE DRAWINGS

Fig. 1 is a transmission electron microscope image of a particle of a composite powder 1.
Fig. 2 is a picture showing Zn mapping of the composite powder 1 by an energy dispersive X-ray spectrometer (hereinafter, EDS) attached to a transmission electron microscope.
Fig. 3 is a picture showing Si mapping of the composite powder 1 by an EDS.
Fig. 4 is a transmission electron microscope image of a particle of a composite powder 8.
Fig. 5 is a picture showing Zn mapping of the composite powder 8 by an EDS.
Fig. 6 is a picture showing Si mapping of the composite powder 8 by an EDS.
Fig. 7 is a transmission electron microscope image of a particle of a composite powder 10.
Fig. 8 is a picture showing Ti mapping of the composite powder 10 by an EDS.
Fig. 9 is a picture showing Si mapping of the composite powder 10 by an EDS.

DETAILED DESCRIPTION OF PREFERRED EMBODIMENTS

Examples of the inorganic powder as a raw material used in the present invention are zinc oxide, titanium oxide, cerium oxide.

For the inorganic powder being ultraviolet blocking particles titanium oxide, zinc oxide, or cerium oxide, the average particle diameter is from 10 to 200 nm. The ultraviolet blocking particles having the particle diameter are used in view of high visible light transparency and a suitable ultraviolet blocking region. If the particle diameter is more than 200 nm, the visible light transparency may be deteriorated, and the ultraviolet blocking performance may be deteriorated. In addition, if the particle diameter is less than 10 nm, the inorganic powder is undesirable in view of the possibility of deteriorating the ultraviolet blocking performance. Further described, when the inorganic powder is not ultraviolet blocking particles, or when the ultraviolet blocking performance is not required, the particle diameter has only to be an optimal size for utilization of the particles. The particle diameter of the inorganic powder is measured by measuring the particle diameters of randomly selected 200 particles with an electron microscope, and calculating the average particle diameter of the primary particles.

The shape of the inorganic powder is not particularly limited, and a powder having any shape can be used, including spherical, rod-shaped, acicularneedle, spindle-shaped, and plate-shaped powders. In rod-shaped, needle-shaped, or spindle-shaped particles, the average particle diameter is defined as the length in the direction of the short axis. In plate-shaped particles, the average particle diameter is defined as the average of the length in the direction of the diagonal line of the plane.

The inorganic powder may be subjected to the surface treatment of the present invention by using any known composite material such as silica surface-treated titanium oxide as a raw material. Further, an oxide doped with a different element such as manganese, calcium, or nitrogen, for example, Ca-doped cerium oxide, may be used as a raw material.

It is significant that a powder having a problem of particularly high surface activity or the elution of ions is used as the inorganic powder to perform the coating of the present invention. In particular, the surface treated amount can be decreased compared to the inactivation by simple silica treatment by applying the present invention to zinc oxide, titanium oxide, or cerium oxide having ultraviolet blocking effect, and therefore the ratio of the inorganic powder in the composite powder can be increased, and the inorganic powder can block ultraviolet light efficiently. Among them, zinc oxide and titanium oxide are preferred.

The inorganic powder of the present invention has a surface treatment layer formed from a water-repellent organic compound on the surface. The surface treatment is a water-repellent treatment in which affinity of the surface of the inorganic powder with water is decreased, and any surface treatment with a material being water-soluble or water-dispersible after the treatment is not included in the "water-repellent treatment" of the present invention. The water-repellent organic compound is a silicone oil. The compound is preferably bound to the inorganic powder via some chemical bond, but even if the compound can physically adsorb the inorganic powder, the compound can provide a certain degree of effect. Described as the water-repellent organic compound but not according to the invention are an alkylsilane, an alkyl titanate, an alkyl aluminate, a polyolefin, a polyester, a metallic soap, an amino acid, and an amino acid salt. The silicone oil is preferred because of its chemical stability. Specific examples of the silicone oil include dimethyl polysiloxane (for example, KF-96A-100cs manufactured by Shin-Etsu Chemical Co., Ltd., and DM10 manufactured by Wacker Asahikasei Silicone Co., Ltd.), methyl hydrogen polysiloxane (for example, KF-99P manufactured by Shin-Etsu Chemical Co., Ltd., and SH1107C manufactured by Dow Corning Toray Co., Ltd.), (dimethicone/methicone)copolymer (for example, KF-9901 manufactured by Shin-Etsu Chemical Co., Ltd.), methyl phenyl silicone (for example, KF-50-100cs manufactured by Shin-Etsu Chemical Co., Ltd.), an amino-modified silicone (for example, KF-8015 manufactured by Shin-Etsu Chemical Co., Ltd., JP-8500 Conditioning Agent manufactured by Dow Corning Toray Co., Ltd., and ADM6060 manufactured by Wacker Asahikasei Silicone Co., Ltd.), triethoxysilylethyl polydimethylsiloxyethyl dimethicone (for example, KF-9908 manufactured by Shin-Etsu Chemical Co., Ltd.), and triethoxysilylethyl polydimethylsiloxyethyl hexyl dimethicone (for example, KF-9909 manufactured by Shin-Etsu Chemical Co., Ltd.). Among them, dimethyl polysiloxane, methyl hydrogen polysiloxane, or a copolymer of these monomers, that is, (dimethicone/methicone) copolymer, is particular preferred in view of low cost, high stability, and strong water repellency due to the simple structure of the polymers. Further, coupling agents such as a silane coupling agent or a titanium coupling agent can be used. The inorganic powder may be surface-treated with at least one compound selected from the water-repellent organic compounds.

The amount of the water-repellent organic compound for treating the inorganic powder is preferably from 2 to 15% by weight based on the amount of the inorganic powder. If the amount is less than 2% by weight, the ion elution amount may be increased because the water repellency becomes insufficient, and the contact between the inorganic powder and water is enhanced. If the amount is more than 15% by weight, cost may be increased, and the effect may plateau.

The composite powder of the present invention further includes a second coating layer containing at least one compound selected from the group consisting of silicon oxide, silicon oxide hydrate, aluminum oxide, and aluminum hydroxide on the surface of the first coating layer formed from the water-repellent organic compound. The second coating layer can give hydrophilicity to the powder, and the powder can be dispersed in water sufficiently.

When the second coating layer contains silicon oxide or silicon oxide hydrate, the amount of the second coating layer is preferably from 5 to 20% by weight in terms of $SiO_2$ based on the amount of the composite powder. If the amount is less than 5% by weight, hydrophilicity of the powder may be insufficient. If the amount is more than 20% by weight, hydrophilicity of the powder may plateau. When the second coating layer contains aluminum oxide or aluminum hydroxide, the amount of aluminum oxide or aluminum hydroxide is preferably from 5 to 20% by weight in terms of $Al_2O_3$ based on the amount of the composite powder. Among them, the material of the second coating layer containing at least one compound selected from the group consisting of silicon oxide, silicon oxide hydrate, aluminum oxide, and aluminum hydroxide is preferably Si oxide or hydroxide which is less reactive than Al oxide or hydroxide.

The second coating layer preferably covers the whole powder uniformly, but the layer does not have to cover the powder completely as long as the resulting composite powder has sufficient hydrophilicity. Further, the composite powder may be pulverized into microparticles by a suitable pulverization treatment as long as the powder maintains its hydrophilicity.

The composite powder of the present invention can be further treated with an oil-based component such as a silicone to give lipophilicity thereto, and dispersed in an oil-based -based medium. Examples of the treatment with the oily component include, but not limited to, for example, a treatment with the water-repellent organic compound as described above.

Preferably, the particle diameter of the composite powder after the covering is 15 μm or less for D90 and 5 μm or less for D50. If the particle diameter is more than the values, the particles aggregate heavily, and a more severe condition is required in order to produce a suitable dispersion condition for use, which is too expensive. From a similar reason, D90 is more preferably 5 μm or less, and D50 is more preferably 3 μm or less. D90 and D50 as described above are values measured according to the method described in the Examples.

Since the composite powder of the present invention includes the two coating layers as described above, the pH of the powder in water does not change for a long time. Specifically, the composite powder preferably has a pH of 6 to 8 when the composite powder is dispersed in water and allowed to stand at 60°C for 2 weeks. The pH of the powder when allowed to stand at 60°C for 2 weeks is the pH value obtained after sufficiently mixing 1 g of the composite powder in 50 mL of pure water in a sealed container, and allowing the composite powder stand in an incubator at 60°C for 2 weeks. The pH is measured with a common pH meter at 20°C.

The method for producing the composite powder of the present invention include, but not limited to, is a method including a step (1) of dispersing an inorganic powder subjected to a surface treatment with a water-repellent organic compound in water; and a step (2) of subjecting the inorganic powder to a surface treatment with a Si source compound and/or an Al source compound, wherein said inorganic powder is titanium oxide, zinc oxide, or cerium oxide, and the average particle diameter thereof is from 10 to 200 nm, and said water-repellent organic compound is a silicone oil. The method for producing the composite powder is also included in the present invention.

Step (1) is a step of dispersing an inorganic powder subjected to a surface treatment with a water-repellent organic compound in a dispersion medium. Examples of the water-repellent organic compound and the inorganic powder include those as described above without limitation. The method of the surface treatment is not particularly limited, and the surface treatment can be performed by any known method. A commercially available powder subjected to a water-repellent treatment can be used. Examples of the commercially available powder subjected to a water-repellent treatment include FINEX-50S-5, FINEX-50S-LP2, FINEX-50-LPT, FINEX-50-LP2, FINEX-50W-LP2 (all manufactured by Sakai Chemical Industry Co., Ltd., zinc oxide subjected to a water-repellent treatment), and STR-100C-LP (manufactured by Sakai Chemical Industry Co., Ltd. , aluminum hydroxide-coated titanium oxide subjected to a water-repellent treatment).

Examples of the dispersion medium used in step (1) include an organic solvent in which the inorganic powder surface-treated with the water-repellent organic compound can be dispersed and the Si source compound and/or the Al source compound can be dissolved, including, but not limited to, alcohols such as isopropanol, and toluene. However, in the present invention, water is preferably used as a dispersion medium in combination with a dispersant. By using the dispersant, the inorganic particles subjected to a water-repellent treatment can be stably dispersed in water without using any organic solvent. Further, it is advantageous in view of the cost, ease of reaction, and safety because no organic solvent is used. Further, in the resulting composite powder, the roughness on the surface thereof can be decreased, the composite powder can have sharp particle size distribution, and the size of the aggregated particle can be decreased. However, use of water is not suitable for a water-soluble inorganic powder.

In step (1), a dispersant can be used in order to disperse the inorganic powder in a dispersion medium uniformly. Examples of the type of the dispersant include, but not limited to, for example, a polycarboxylic acid or a salt thereof, an alkyl sulfonic acid or a salt thereof, an alkyl benzene sulfonic acid or a salt thereof, a naphthalenesulfonic acid or a salt thereof, a polyether alkyl sulfonic acid or a salt thereof, an alkylbetaine, a polyether or a derivative thereof, a polyether alkyl ether, a polyoxyalkylene alkenyl phenyl ether, a sorbitan fatty acid ester, a polyether sorbitan fatty acid ester, a polyether fatty acid ester, a glycerin fatty acid ester, a polyether hydrogenated castor oil, a polyetheralkylamine, a polyether-modified silicone, a polyglycerin-modified silicone, and an alcohol. Although any one of an anionic, cationic, and nonionic dispersants can be used, a nonionic dispersant is preferred in order to prevent the chemical reaction with the inorganic powder. The dispersion may be used as a combination of two or more dispersions. In step (2), when alkaline Na silicate is used as a Si source compound, or heating is required, a polyether-modified silicone is preferably used.

Further, when water is used as a dispersion medium, the dispersant preferably has an HLB value of 10 to 17. If the HLB value is out of the range, the powder may be dispersed insufficiently, and various physical properties such as ultraviolet blocking performance may be affected. In the present invention, the HLB value is calculated by the following expression as defined by W. C. Grifinn:

$$N_{HLB} = (E + P)/5$$

($N_{HLB}$: HLB value, E: % by weight of a polyoxyethylene moiety based on the whole molecule of the dispersant, P: % by weight of a polyhydric alcohol moiety based on the whole molecules of the dispersant)

The amount of the dispersant is preferably from 2 to 15% by weight based on the total amount of the dispersion obtained in step (1). In particular, when water is used as a solvent, if the amount is less than the range, the powder may become hard to blend in water. If the amount is more than the range, it is disadvantages in cost, or a lot of bubbles may be produced.

The dispersant may remain in the composite powder as long as the dispersant does not influence adversely on the inactivation or use of the composite powder of the present invention.

A method for dispersing the composite powder in step (1) may be any known method. For example, a method using a bead mill or a high-pressure homogenizer is suitable.

Step (2) is a step of subjecting the inorganic powder to a surface treatment with a Si source compound and/or an Al source compound. Examples of the surface treatment include, but not limited to, a wet process in which a Si source compound such as tetraalkoxysilane or sodium silicate is made to present in the dispersion obtained in step (1), and $SiO_2$ is precipitated on the surface of the powder by hydrolysis or thermolysis. Since the wet process can facilitate precipitation of $SiO_2$ on the surface of the inorganic powder and precipitate $SiO_2$ uniformly compared to a dry process, the aggregation of the powder after the treatment can be prevented. Preferred examples of the Si source compound and/or the Al source compound include compounds which easily convert to $SiO_2$, $Al(OH)_3$, or $Al_2O_3$, including a tetraalkoxysilane or a hydrolysis condensate thereof, sodium silicate, potassium silicate, an aluminum alkoxide or a hydrolysis condensate thereof, and sodium aluminate. Two or more compounds may be used together.

Examples of the hydrolysis include, but not limited to, a method using an acid such as sulfuric acid, hydrochloric acid, acetic acid, or nitric acid. The method for neutralizing in the method for treatment of the aqueous dispersion with silica may be a method of adding an acid to the dispersion, and then adding a Si source compound and/or an Al source compound, a method of adding a Si source compound and/or an Al source compound to the dispersion, and then adding an acid, or a method of adding a Si source compound and/or an Al source compound and an acid to the dispersion simultaneously.

In the composite powder of the present invention, the hydrophilicity is given on the surface of the composite powder to improve dispersibility in water by the functuion of the Si source compound and/or the Al source compound, and the water-repellent organic compound prevents the inorganic powder as a core of the composite powder from directly contacting with water by barriering water. Therefore, the composite powder is stably dispersed in an aqueous medium, and the elution of ions over time is remarkably small. Since the core inorganic powder is covered with the hydrophilic second coating layer and the water-repellent first coating layer, the core inorganic powder is not contacted with both a hydrophilic material and a lipophilic material, and the surface activity can be suppressed. In particular, in the inorganic powder from which a trace amount of metal ions are eluted, such as zinc oxide, it is advantageous in that the pH in the formulation is stabilized.

The composite powder thus obtained may be post-treated, for example, filtrated, washed with water, or dried, if needed. Further, the composite powder may be classified by a screen, if needed. Examples of the classification by a screen include wet classification and dry classification.

The composite powder of the present invention can also be mixed in cosmetics, sunscreen inks, coatings, or resin compositions. In particular, an aqueous cosmetic having good stability and ultraviolet blocking effect can be obtained because of the properties as described above. Further, the composite powder of the present invention can be used as a raw material by dispersing in water or an oil. The present invention also provides a cosmetic containing the composite powder as described above.

The cosmetic is not particularly limited. Cosmetics for ultraviolet prevention such as a sunscreen agent; cosmetics for base make up such as a foundation; and cosmetics for point make up such as a lipstick can be obtained by mixing the composite powder of the present invention with any cosmetic raw material, if needed.

The cosmetic can be in any form, for example, a form of an oil-based cosmetic, a water-based cosmetic, an O/W type cosmetic, or a W/O type cosmetic. Among them, the composite powder can be suitably used in sunscreen agents.

The cosmetic may contain any water-based component or an oil-based component which can be used in the cosmetic field. The water-based component and the oil-based component may contain any component, including, but not limited to, for example, an oil solution, a surfactant, a humectant, a higher alcohol, a sequestering agent, a natural or synthetic polymer, a water-soluble or oil-soluble polymer, an ultraviolet blocking agent, various extracts, a coloring agent such as an organic dye, a preservative, an antioxidant, a colorant, a thickener, a pH adjuster, a perfume, a cooling-sensation agent, an antiperspirant, a bactericidal agent, a skin activating agent, and various powders.

Examples of the oil solution include, but not limited to, for example, natural animal and plant fats (for example, olive oil, mink oil, castor oil, palm oil, beef tallow, evening primrose oil, coconut oil, castor oil, cacao oil, and macadamia nut oil); waxes (for example, jojoba oil, beeswax, lanolin, carnauba wax, and candelilla wax) ; higher alcohols (for example, lauryl alcohol, stearyl alcohol, cetyl alcohol, and oleyl alcohol); higher fatty acids (for example, lauric acid, palmitic acid, stearic acid, oleic acid, behenic acid, and lanolin fatty acid) ; higher aliphatic hydrocarbons (for example, liquid paraffin, solid paraffin, squalane, vaseline, ceresin, and microcrystalline wax); synthetic ester oils (for example, butyl stearate, hexyl laurate, diisopropyl adipate, diisopropyl sebacate, octyldodecyl myristate, isopropyl myristate, isopropyl palmitate, isopropyl myristate, cetyl isooctanoate, and neopentyl glycol dicaprate); and silicone derivatives (for example, silicone oils such as methyl silicone and methyl phenyl silicone) . Further, an oil-soluble vitamin, a preservative, or a whitening agent may be blended.

Examples of the surfactant include a lipophilic nonionic surfactant and a hydrophilic nonionic surfactant. Examples of the lipophilic nonionic surfactant include, but not limited to, for example, sorbitan monooleate, sorbitan monoisostearate, sorbitan monolaurate, sorbitan monopalmitate, sorbitan monostearate, sorbitan sesquioleate, sorbitan trioleate, sorbitan fatty acid esters such as diglycerol sorbitan penta-2-ethylhexylate and diglycerol sorbitan tetra-2-ethylhexylate, glycerin fatty acids such as glycerol mono-cottonseed oil fatty acid, glycerol monoerucate, glycerol sesquioleate, glycerol monostearate, $\alpha,\alpha'$- glycerol oleate pyroglutamate, and glycerol monostearate malate; propylene glycol fatty acid esters such as propylene glycol monostearate; hydrogenated castor oil derivatives; and glycerol alkyl ethers.

Examples of the hydrophilic nonionic surfactant include, but not limited to, for example, POE sorbitan fatty acid esters such as POE sorbitan monooleate, POE sorbitan monostearate, and POE sorbitan tetraoleate; POE sorbit fatty acid esters such as POE sorbit monolaurate, POE sorbit monooleate, POE sorbit pentaoleate, and POE sorbit monostearate; POE glycerin fatty acid esters such as POE glycerin monostearate, POE glycerin monoisostearate, and POE glycerin triisostearate; POE fatty acid esters such as POE monooleate, POE distearate, POE monodioleate, and distearic acid ethylene glycol; POE alkyl ethers such as POE lauryl ether, POE oleyl ether, POE stearyl ether, POE behenyl ether, POE 2-octyl dodecyl ether, and POE cholestanol ether; POE alkyl phenyl ethers such as POE octyl phenyl ether, POE nonyl phenyl ether, and POE dinonyl phenyl ether; Pluaronic types such as Pluronic; POE/POP alkyl ethers such as POE/POP cetyl ether, POE/POP2-decyl tetradecyl ether, POE/POP monobutyl ether, POE/POP hydrogenated lanolin, and POE/POP glycerin ether; tetra POE/tetra POP ethylenediamine condensation products such as Tetronic; POE castor oil hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamic acid monoisostearic acid diester, and POE hydrogenated castor oil maleic acid; POE beeswax/lanolin derivatives such as POE sorbit beeswax; alkanolamides such as coconut oil fatty acid diethanolamide, lauric acid mono ethanolamide, and fatty acid isopropanolamide; POE propylene glycol fatty acid esters, POE alkylamines, POE fatty acid amides, sucrose fatty acid esters, POE nonyl phenyl formaldehyde condensation products, alkyl ethoxydimethylamine oxides, and trioleyl phosphates.

Any other surfactant may be blended, including, for example, anionic surfactants such as fatty acid soaps, higher alkyl sulfate ester salts, POE lauryl sulfate triethanolamine, and alkyl ether sulfate ester salts; cationic surfactants such as alkyl trimethyl ammonium salts, alkyl pyridinium salts, alkyl quaternized ammonium salts, alkyl dimethyl benzylammonium salts, POE alkylamines, alkylamine salts, and polyamine fatty acid derivatives; and amphoteric surfactants such as an imidazoline-based amphoteric surfactant and a betaine-based surfactant, as long as the surfactant does not affect the stability and skin irritation.

Examples of the humectant include, but not limited to, for example, xylitol, sorbitol, maltitol, chondroitin sulfate, hyaluronic acid, mucoitinsulfuric acid, caronic acid, atelocollagen, cholesteryl-12-hydroxystearate, sodium lactate, bile salt, dl-pyrrolidone carboxylate salts, short chain soluble collagen, (EO)PO adducts of diglycerin, Rosa Roxburghii Fruit extract, yarrow extract, and melilot extract.

Examples of the higher alcohol include, but not limited to, for example, linear alcohols such as lauryl alcohol, cetyl alcohol, stearyl alcohol, behenyl alcohol, myristyl alcohol, oleyl alcohol, and cetostearyl alcohol; and branched alcohols such as monostearyl glycerin ether (batyl alcohol), 2-decyl tetradecinol, lanolin alcohol, cholesterol, phytosterol, hexyldodecanol, isostearyl alcohol, and octyl dodecanol.

Examples of the sequestering agent include, but not limited to, for example, 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, and edetic acid.

Examples of the natural water-soluble polymer include, but not limited to, for example, plant polymers such as gum arabic, tragacanth gum, galactan, guar gum, carob gum, karaya gum, carrageenan, pectin, agar, quince seed (Cydonia oblonga), algae colloid (brown alga extract), starch (rice, corn, potato, wheat), and glycyrrhizinic acid; microbial polymers such as xanthan gum, dextran, succinoglycan, and pullulan; and animal polymers such as collagen, casein, albumin, and gelatin.

Examples of the semisynthetic water-soluble polymer include, but not limited to, for example, starch polymers such as carboxymethyl starch and methyl hydroxypropyl starch; cellulose polymers such as methylcellulose, nitrocellulose, ethylcellulose, methyl hydroxypropyl cellulose, hydroxyethyl cellulose, cellulose sodium sulfate, hydroxypropyl cellulose, sodium carboxymethyl cellulose (CMC), crystalline cellulose, and cellulose powder; and alginate polymers such as sodium alginate and alginic acid propylene glycol ester.

Examples of the synthetic water-soluble polymer include, but not limited to, for example, vinyl polymers such as polyvinyl alcohol, polyvinyl methyl ether, and polyvinylpyrrolidone; polyoxyethylene polymers such as polyethylene glycol 20,000, 40,000, and 60,000; copolymers such as polyoxyethylene polyoxypropylene copolymer; acrylic polymers such as sodium polyacrylate, polyethyl acrylate, and polyacrylamide; polyethylenimine, cationic polymer, carboxyvinyl polymer, and alkyl-modified carboxyvinyl polymer.

Examples of the inorganic water-soluble polymer include, but not limited to, for example, bentonite, magnesium aluminum silicate (Veegum), laponite, hectorite, and silicic anhydride.

Examples of the ultraviolet blocking agent include, but not limited to, for example, benzoic acid-based ultraviolet blocking agents such as p-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, and N,N-dimethyl PABA butyl ester; anthranilic acid-based ultraviolet blocking agents such as homomenthyl-N-acetyl anthranilate; salicylic acid-based ultraviolet blocking agents such as amyl salicylate, menthyl salicylate, homomenthyl salicylate, octyl salicylate, phenyl salicylate, benzyl salicylate, and p-isopropanol phenyl salicylate; cinnamic acid-based ultraviolet blocking agents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate, and glyceryl mono-2-ethylhexanoyl-diparamethoxycinnamate; benzophenone-based ultraviolet blocking agents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methyl benzophenone, 2-hydroxy-4-methoxybenzophenone-5-sulfonate, 4-phenyl ben-

EP 2 740 771 B1

zophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methyl benzylidene)-d,1-camphor, 3-benzylidene-d,1-camphor, urocanic acid, urocanic acid ethyl ester, 2-phenyl-5-methyl benzoxazole, 2,2'-hydroxy-5-methyl phenyl benzotriazole, 2-(2'-hydroxy-5'-t-octylphenyl)benzotriazole, 2-(2'-hydroxy-5'-methylphenyl benzotriazole, dibenzalazine, dianisoylmethane, 4-methoxy-4'-t-butyldibenzoylmethane, and 5-(3,3-dimethyl-2-norbornylidene)-3-pentan-2-one.

Examples of the other chemical component include, but not limited to, for example, vitamins such as vitamin A oil, retinol, retinol palmitate, inosit, pyridoxine hydrochloride, benzyl nicotinate, nicotinamide, DL-$\alpha$-tocopherol nicotinate, magnesium ascorbyl phosphate, 2-O-$\alpha$-D-glucopyranosyl-L-ascorbic acid, vitamin D2 (ergocalciferol), dl-$\alpha$-tocopherol, DL-($\alpha$-tocopherol acetate, pantothenic acid, and biotin; hormones such as estradiol and ethinyl estradiol; amino acids such as arginine, aspartic acid, cystine, cysteine, methionine, serine, leucine, and triptophan; anti-inflammatory agents such as allantoin and azulene; whitening agents such as arbutin; astringents such as tannic acid; refrigerants such as L-menthol and camphor; sulfur, lysozyme chloride, and pyridoxine chloride.

Examples of various extracts include, but not limited to, for example, Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, and licorice extract.

Examples of various powders include luster color pigments such as red iron oxide, yellow iron oxide, black iron oxide, mica titanium, iron oxide-coated mica titanium, and titanium oxide-coated glass flake; inorganic powders such as mica, talc, kaolin, and sericite; and organic powders such as polyethylene powder, nylon powder, crosslinked polystyrene, cellulose powder, and silicone powder. Preferably, some or all of powder components are hydrophobized with a material such as a silicone, a fluorine compound, a metallic soap, an oil solution, or an acyl glutamic acid salt by a known method in order to improve sensory characteristics and makeup retainability. Further, a composite powder other than the powder of the present invention may be blended and used.

When the composite particles of the present invention are used as a component added to a sunscreen ink, examples of the pigment include colored pigments such as titanium oxide, red iron oxide, antimony red, cadmium yellow, cobalt blue, prussian blue, ultramarine, carbon black, and graphite; and extender pigments such as calcium carbonate, kaolin, clay, barium sulfate, aluminum hydroxide, and talc. Further, the composite particles of the present invention can be used with the organic pigment including pigment components such as a soluble azo pigment, an insoluble azo pigment, an azo lake pigment, a condensed azo pigment, a copper phthalocyanine pigment, and a condensed polycyclic pigment; binder resins such as a shellac resin, an acrylic resin, a styrene-acrylic resin, a styrene-maleic acid resin, a styrene-acrylic-maleic acid resin, a polyurethane resin, a polyester resin, and a polyamide resin; and water-miscible organic solvents.

When the composite particles of the present invention are blended in a coating, a resin in the coating may be a curable or uncurable resin. The coating may be a solvent-based coating containing an organic solvent, or an aqueous coating in which a resin is dissolved or dispersed in water.

When the composite particles of the present invention are used as a component added to a coating composition, the particles can be used with film-forming resins such as an acrylic resin, a polyester resin, and an epoxy resin; various pigments such as a colored pigment, a extender pigment, and a luster pigment; a curing catalyst, a surface control agent, an antifoaming agent, a pigment dispersant, a plasticizer, a film-forming aid, an ultraviolet absorbing agent, an antioxidant, and the like.

The composite powder of the present invention can be mixed in a resin and used as a resin composition. In this case, the resin used may be a thermoplastic resin or a thermosetting resin. Examples of the resin include an epoxy resin, a phenol resin, a polyphenylene sulfide (PPS) resin, a polyester-based resin, a polyamide, a polyimide, a polystyrene, a polyethylene, a polypropylene, polyvinyl chloride, polyvinylidene chloride, a fluorine resin, polymethyl methacrylate, an ethylene-ethyl acrylate copolymer (EEA) resin, a polycarbonate, a polyurethane, a polyacetal, a polyphenylene ether, a polyetherimide, an acrylonitrile-butadiene-styrene copolymer (ABS) resin, a liquid crystal resin (LCP), a silicone resin, and an acrylic resin.

EXAMPLES

While the present invention will now be described in more detail with reference to the examples, the present invention is not limited to these examples.

(Example 1)

To 500 mL of pure water was added 20 g of a polyether-modified silicone (KF-6011: manufactured by Shin-Etsu Chemical Co., Ltd. : HLB 14.5), and 100 g of zinc oxide treated with 4% hydrogen dimethicone (FINEX-50S-LP2) was further added, mixed and dispersed. To the dispersion liquid was added 27.5 mL of an aqueous Na silicate solution ($SiO_2$: 403.9 g/L, $Na_2O$: 130.7 g/L) (11.1 g as the amount of $SiO_2$, 10% by weight based on the total amount of the powder after treatment), and stirred for 20 minutes or more. After that, the temperature of the liquid was changed to 70°C. A certain amount of dilute sulfuric acid was added dropwise to the solution to neutralize the solution. During the neutralization, the amount of sulfuric acid was adjusted so that the condition of pH 8.5 or more was maintained for 90 minutes or more, and the neutralization was continued until the pH was changed to 6.5 ± 0.5. After the completion of the neutralization, the liquid was aged for 1 hour while the liquid temperature was kept at 70°C. After that, the liquid was filtered, and washed with water. The cake obtained by washing with water was dried to obtain a composite powder 1.

(Example 2)

A composite powder 2 was obtained by the same method as in Example 1, except that zinc oxide treated with 7% hydrogen dimethicone (FINEX-50-LPT: manufactured by Sakai Chemical Industry Co., Ltd.) was used in place of zinc oxide treated with 4% hydrogen dimethicone (FINEX-50S-LP2).

(Example 3)

To 500 mL of pure water was added 20 g of a polyether-modified silicone (KF-6011: manufactured by Shin-Etsu Chemical Co., Ltd. : HLB 14.5) , and 100 g of zinc oxide treated with 7% hydrogen dimethicone (FINEX-50-LPT) was further added, stirred and dispersed. After the dispersion liquid was heated to 70°C, a separate container including 27.5 mL of an aqueous Na silicate solution (the amount of $SiO_2$ in the solution is 11.1 g, 10% by weight based on the total amount of the powder after treatment) and a separate container including dilute sulfuric acid were provided. Each solution in the two separate containers was added dropwize to the dispersion liquid. During the addition, the amount was adjusted so that the pH was maintained at a constant value within the range of 5.5 to 7.5, and so that the addition was completed 2 hours later. After the completion of the addition, the liquid was aged for 1 hour while the liquid temperature was kept at 70°C. After that, the liquid was filtered, and washed with water. The cake obtained by washing with water was dried to obtain a composite powder 3.

(Example 4)

A composite powder 4 was obtained by the same method as in Example 3, except that the amount of the aqueous Na silicate solution was changed to 13.1 mL (the amount of $SiO_2$ in the solution is 5.3 g, 5% by weight based on the total amount of the powder after treatment).

(Example 5)

A composite powder 5 was obtained by the same method as in Example 3, except that zinc oxide (primary particle diameter 20 nm) treated with 12% hydrogen dimethicone was used in place of zinc oxide treated with 7% hydrogen dimethicone (FINEX-50-LPT) .

(Example 6) (reference example)

A composite powder 6 was obtained by the same method as in Example 3, except that zinc oxide (primary particle diameter 20 nm) treated with 4% titanium coupling agent (PLENACT KR TTS: manufactured by Ajinomoto Fine-Techno Co., Inc.) was used in place of zinc oxide treated with 8% hydrogen dimethicone (FINEX-50-LPT) .

(Example 7) (reference example)

A composite powder 7 was obtained by the same method as in Example 3, except that zinc oxide (primary particle diameter 20 nm) treated with 4% caprylyl silane (AES-3083: manufactured by Shin-Etsu Chemical Co., Ltd.) was used in place of zinc oxide treated with 8% hydrogen dimethicone (FINEX-50-LPT).

(Example 8)

To 30 g of zinc oxide treated with 4% by weight of hydrogen dimethicone (FINEX-50S-LP2) were added 5.5 g of tetraethylsilane (1.6 g as the amount of $SiO_2$, 5% by weight based on the total amount of the powder after treatment) and 30 mL of isopropanol, and mixed sufficiently. To the mixture was added a few drops of aqueous ammonia, and then mixed. The mixture was dried at 120°C to obtain a composite powder 8.

(Example 9)

A composite powder 9 was obtained by the same method as in Example 8, except that the amount of tetraethylsilane was changed to 7.8 g (2.3 g as the amount of $SiO_2$, 7% by weight based on the total amount of the powder after treatment).

(Example 10)

To 500 mL of pure water was added 20 g of a polyether-modified silicone (KF-6011: manufactured by Shin-Etsu Chemical Co., Ltd.: HLB 14.5), and 100 g of titanium oxide treated with 7% aluminium hydroxide and 4% hydrogen dimethicone (STR-100C-LP: manufactured by Sakai Chemical Industry Co., Ltd.) was further added, mixed and dispersed. To the dispersion liquid was added 27.5 mL of sodium silicate ($SiO_2$: 403.9 g/L, $Na_2O$: 130.7 g/L) (11.1 g as the amount of $SiO_2$, 10% by weight based on the total amount of the powder after treatment), and stirred for 20 minutes or more. After that, the temperature of the liquid was changed to 70°C. A certain amount of dilute sulfuric acid was added dropwise to the solution to neutralize the solution. During the neutralization, the amount of sulfuric acid was adjusted so that the condition of pH 8.5 or more was maintained for 90 minutes or more, and the neutralization was continued until the pH was changed to 6.5 ± 0.5. After the completion of the neutralization, the liquid was aged for 1 hour while the liquid temperature was kept at 70°C. After that, the liquid was filtered, and washed with water. The cake obtained by washing with water was dried to obtain a composite powder 10.

(Comparative Example 1)

A composite powder 11 was obtained by the same method as in Example 1, except that the amount of the aqueous Na silicate solution was changed to 5.0 mL (the amount of $SiO_2$ in the solution is 2.0 g, 2% by weight based on the total amount of the powder after treatment).

(Comparative Example 2)

To 500 mL of pure water was added 100 g of zinc oxide without being covered with a water-repellent organic compound (FINEX-50: primary particle diameter 20 nm), and dispersed. To the dispersion liquid was added 27.5 mL of an aqueous Na silicate solution ($SiO_2$: 403. 9 g/L, $Na_2O$: 130.7 g/L) (11.1 g as the amount of $SiO_2$), and stirred for 20 minutes or more. After that, the temperature of the liquid was changed to 70°C. A certain amount of dilute sulfuric acid was added dropwise to the solution to neutralize the solution. During the neutralization, the amount of sulfuric acid was adjusted so that the condition of pH 8.5 or more was maintained for 90 minutes or more, and the neutralization was continued until the pH was changed to 6.5 ± 0.5. After the completion of the neutralization, the liquid was aged for 1 hour while the liquid temperature was kept at 70°C. After that, the liquid was filtered, and washed with water. The cake obtained by washing with water was dried to obtain a composite powder 12.

(Comparative Example 3)

A composite powder 13 was obtained by the same method as in Comparative Example 2, except that the amount of the aqueous Na silicate solution was changed to 61.9 mL (25 g as the amount of $SiO_2$, 20% by weight based on the total amount of the powder after treatment).

(Comparative Example 4)

Zinc oxide microparticles having a primary particle diameter of 20 nm (FINEX-50) were named as a powder 14.
For the resulting composite powders, the results of water repellency and texture when touched with a hand are shown in Table 1. The water repellency was tested by adding 0.5 g of the powder to 100 mL of water, and then mixing and observing the resultant 30 minutes later. When the composite powder was present on water, it was determined that the composite powder had water repellency. The evaluation criteria of roughness are as follows. The composite powder was rubbed with two fingers, and if any hard particle remained unbroken, it was determined that the composite powder had a rough texture. The composite powders 8 and 9 had a rough texture, suggesting that the silica layer of the outermost layer is not homogeneous, and some particles aggregate. Because the powders have hydrophilicity even though the powders have a rough texture, the powders have no problem in use although they require much time for the dispersion treatment.

[Table 1]

|  | Water repellency | Rough texture |
| --- | --- | --- |
| Example |  |  |
| Composite powder 1 | No | No |
| Composite powder 2 | No | No |
| Composite powder 3 | No | No |
| Composite powder 4 | No | No |
| Composite powder 5 | No | No |
| Composite powder 6 | No | No |
| Composite powder 7 | No | No |
| Composite powder 8 | No | Yes |
| Composite powder 9 | No | Yes |
| Composite powder 10 | No | No |
| Comparative example |  |  |
| Composite powder 11 | Yes | No |
| Composite powder 12 | No | No |
| Composite powder 13 | No | No |
| Powder 14 | No | No |

Further, the results of the particle size distribution of the resulting composite powders are shown in Table 2. The particle size distribution was measured with a laser diffraction particle size distribution measuring machine (LA-750: manufactured by HORIBA, Ltd.) by adding 0.5 g of a sample to 100 g of a 0.025% by weight aqueous solution of sodium hexametaphosphate, and dispersing the mixture with an ultrasonic homogenizer for 3 minutes.

[Table 2]

|  | Particle size distribution D10 ($\mu$m) | Particle size distribution D50 ($\mu$m) | Particle size distribution D90 ($\mu$m) |
| --- | --- | --- | --- |
| Composite powder 1 | 0.9360 | 1.3814 | 1.9586 |
| Composite powder 2 | 1.5366 | 2.6003 | 4.6862 |
| Composite powder 5 | 0.8308 | 1.2947 | 1.8174 |
| Composite powder 8 | 0.2448 | 3.5030 | 13.1545 |

Table 2 shows that in the composite powders obtained by the production method of the present invention, a composite powder produced by the method using water as a dispersion medium had a sharp particle size distribution and a small size of the aggregated particles. Therefore, it is found that the composite powder is advantageous in terms of the dispersion treatment that is performed for mixing the composite powder in coatings, cosmetics, and sunscreens.

The powders were stored in water for a long time. The results of the change of pH thereof are shown in Table 3. The pH (initial pH) was measured by charging 50 mL of pure water and 1 g of each sample in a mayonnaise bottle, mixing them sufficiently, and opening the bottle for measurement. After the bottle was sealed again, the bottle was stood in an incubator at 60°C for 2 weeks, and then the pH was measured.

[Table 3]

| | Type of organic treatment | Amount of organic treatment (% by weight based on the amount of inorganic powder) | Amount of inorganic silica (% by weight based on the amount of whole powder) | Initial pH | pH after 2 weeks |
|---|---|---|---|---|---|
| Composite powder 1 | Hydrogen dimethicone | 4 | 10 | 7.15 | 7.58 |
| Composite powder 2 | Hydrogen dimethicone | 7 | 10 | 7.19 | 7.05 |
| Composite powder 3 | Hydrogen dimethicone | 7 | 10 | 6.75 | 7.37 |
| Composite powder 4 | Hydrogen dimethicone | 7 | 5 | 7.74 | 7.79 |
| Composite powder 5 | Hydrogen dimethicone | 12 | 10 | 6.68 | 6.91 |
| Composite powder 6 | Titanium coupling agent | 4 | 10 | 6.96 | 7.89 |
| Composite powder 7 | Caprylyl silane | 4 | 10 | 7.63 | 7.97 |
| Composite powder 8 | Hydrogen dimethicone | 4 | 5 | 7.22 | 7.89 |
| Composite powder 9 | Hydrogen dimethicone | 4 | 7 | 7.31 | 7.78 |
| Composite powder 10 | Hydrogen dimethicone | 4 | 10 | 6.81 | 6.95 |
| Composite powder 11 | Hydrogen dimethicone | 4 | 2 | Unmeasurable | Unmeasurable |
| Composite powder 12 | - | - | 10 | 8.01 | 9.18 |
| Composite powder 13 | - | - | 20 | 7.89 | 8.88 |
| Powder 14 | - | - | - | 7.75 | 9.21 |

Table 3 shows that aqueous dispersions containing the composite powder of the present invention were stable, and the changes of the pH over time were small.

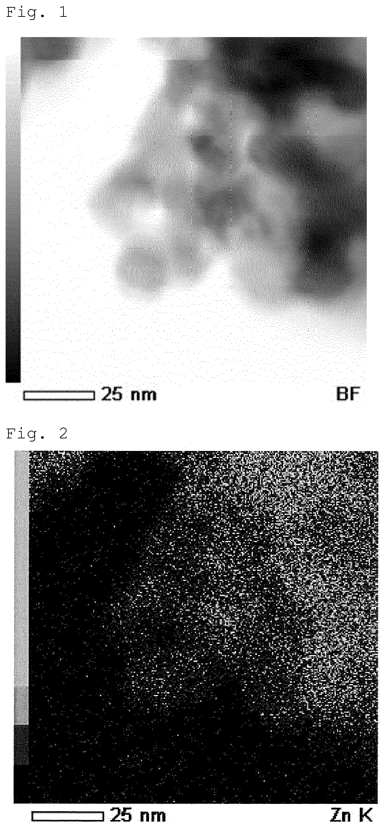

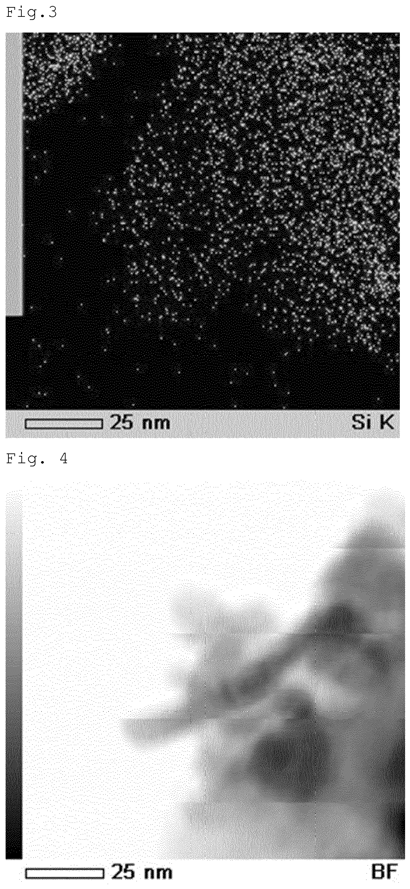

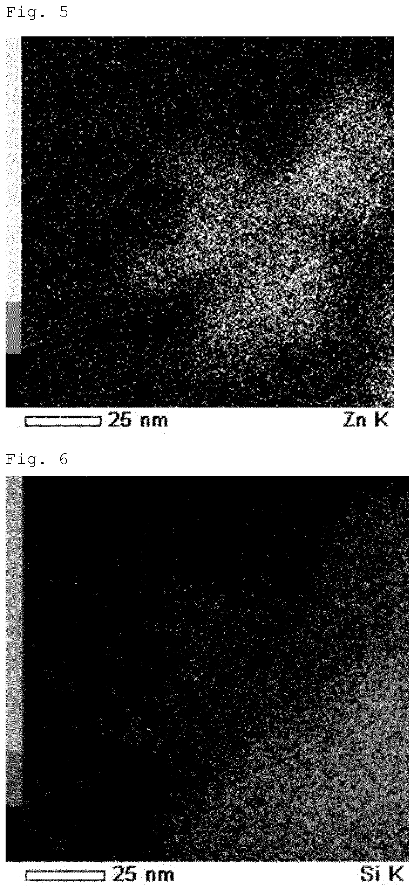

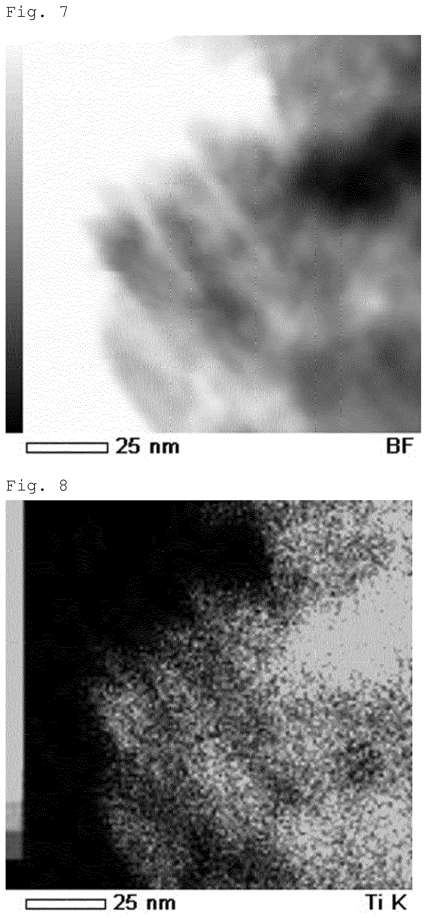

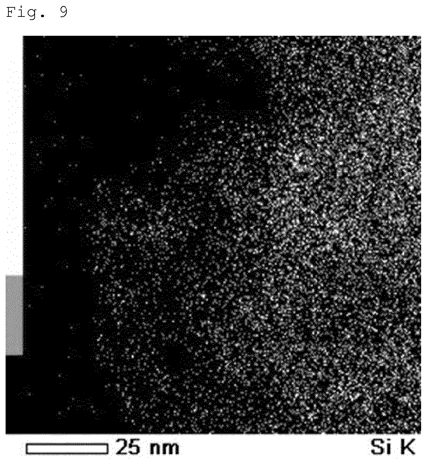

Images of particles of the composite powder 1, the composite powder 8, and the composite powder 10 were taken by a transmission electron microscope, and Zn, Ti, and Si element mapping analysis was performed with an energy dispersive X-ray spectrometer attached to a transmission electron microscope. Figs. 1, 4, and 7 are each a transmission electron microscope image. Figs. 2 and 5 are each a picture of Zn mapping. Fig. 8 is a picture of Ti mapping. In the pictures, points where a Zn atom or a Ti atom was detected are shown as white dots. Similarly, Figs. 3, 6, and 9 are each a picture of Si mapping, in which points where a Si atom was detected are shown as white dots. The result confirmed that the composite powder obtained by the production method of the present invention was covered with silica. The results also showed that in the composite powders obtained by the production method of the present invention, in the composite powder 1 and the composite powder 10 produced by the method using water as a dispersion medium, the points of mapping of titanium oxide and silica coincided with each other well (Figs. 2 and 3, Figs. 8 and 9), showing that the composite powders are treated particularly uniformly.

INDUSTRIAL APPLICABILITY

The composite powder of the present invention can be used as a component added to plastics, inks, and cosmetics.

**Claims**

1. A composite powder, comprising:

   an inorganic powder;
   a first coating layer containing a water-repellent organic compound on the surface of the inorganic powder; and
   a second coating layer which contains at least one compound selected from the group consisting of silicon oxide, silicon oxide hydrate, aluminum oxide, and aluminum hydroxide, and
   which is formed on the first coating layer, wherein
   said inorganic powder is titanium oxide, zinc oxide, or
   cerium oxide, and the average particle diameter thereof is from 10 to 200 nm, and said water-repellent organic compound is a silicone oil.

2. The composite powder according to claim 1, wherein the inorganic powder is an inorganic powder having an ultraviolet blocking effect.

3. A method for producing the composite powder according to claim 1 or 2, comprising the steps of:

   (1) dispersing an inorganic powder subjected to a surface treatment with a water-repellent organic compound in a dispersion medium; and
   (2) subjecting the inorganic powder to a surface treatment with a Si source compound and/or an Al source compound,

   wherein said inorganic powder is titanium oxide, zinc oxide, or cerium oxide, and the average particle diameter thereof is from 10 to 200 nm, and said water-repellent organic compound is a silicone oil.

4. The method for producing the composite powder according to claim 3, wherein the dispersion medium comprises water.

5. A cosmetic, comprising the composite powder according to claim 1 or 2.


**Patentansprüche**

1. Verbundpulver, umfassend:

   ein anorganisches Pulver;
   eine erste Überzugsschicht, die eine wasserabweisende organische Verbindung umfasst, auf der Oberfläche des anorganischen Pulvers; und
   eine zweite Überzugsschicht, die mindestens eine Verbindung, ausgewählt aus der Gruppe bestehend aus Siliciumoxid, Siliciumoxid-Hydrat, Aluminiumoxid und Aluminiumhydroxid, umfasst und die auf der ersten Überzugsschicht gebildet ist, wobei
   das anorganische Pulver Titanoxid, Zinkoxid oder Ceroxid ist und der mittlere Teilchendurchmesser davon von 10 bis 200 nm beträgt und die wasserabweisende organische Verbindung ein Silikonöl ist.

2. Verbundpulver nach Anspruch 1, wobei das anorganische Pulver ein anorganisches Pulver mit einer UV-blockierenden Wirkung ist.

3. Verfahren zur Herstellung des Verbundpulvers nach Anspruch 1 oder 2, umfassend die folgenden Schritte:

(1) Dispergieren eines einer Oberflächenbehandlung mit einer wasserabweisenden organischen Verbindung unterzogenen anorganischen Pulvers in einem Dispersionsmedium; und

(2) Unterziehen des anorganischen Pulvers einer Oberflächenbehandlung mit einer Verbindung als Si-Quelle und/oder einer Verbindung als Al-Quelle,

wobei das anorganische Pulver Titanoxid, Zinkoxid oder Ceroxid ist und der mittlere Teilchendurchmesser davon von 10 bis 200 nm beträgt und die wasserabweisende organische Verbindung ein Silikonöl ist.

4. Verfahren zur Herstellung des Verbundpulvers nach Anspruch 3, wobei das Dispersionsmedium Wasser umfasst.

5. Kosmetikum, umfassend das Verbundpulver nach Anspruch 1 oder 2.

**Revendications**

1. Poudre composite comprenant :

une poudre inorganique ;

une première couche de revêtement contenant un composé organique hydrofuge sur la surface de la poudre inorganique ; et

une deuxième couche de revêtement qui contient au moins un composé choisi dans le groupe constitué de l'oxyde de silicium, de l'oxyde de silicium hydraté, de l'oxyde d'aluminium, et de l'hydroxyde d'aluminium, et qui est formée sur la première couche de revêtement, ladite poudre inorganique étant de l'oxyde de titane, de l'oxyde de zinc, ou de l'oxyde de cérium, et le diamètre particulaire moyen de celui-ci étant de 10 à 200 nm, et ledit composé organique hydrofuge étant une huile silicone.

2. Poudre composite selon la revendication 1, ladite poudre inorganique étant une poudre inorganique possédant un effet de blocage des ultraviolets.

3. Procédé de production de la poudre composite selon la revendication 1 ou la revendication 2, comprenant les étapes de :

(1) dispersion d'une poudre inorganique soumise à un traitement de surface avec un composé organique hydrofuge dans un milieu de dispersion ; et

(2) soumission de la poudre inorganique à un traitement de surface avec un composé source de Si et/ou un composé source d'Al,

dans lequel ladite poudre inorganique est de l'oxyde de titane, de l'oxyde de zinc, ou de l'oxyde de cérium, et le diamètre particulaire moyen de celle-ci est de 10 à 200 nm, et ledit composé organique hydrofuge est une huile silicone.

4. Procédé de production de la poudre composite selon la revendication 3, dans lequel le milieu de dispersion comprend de l'eau.

5. Cosmétique, comprenant la poudre composite selon la revendication 1 ou la revendication 2.

Fig. 1

25 nm      BF

Fig. 2

25 nm      Zn K

Fig.3

Fig. 4

Fig. 5

25 nm      Zn K

Fig. 6

25 nm      Si K

Fig. 7

25 nm                                          BF

Fig. 8

25 nm                                          Ti K

Fig. 9

25 nm                                    Si K

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006034879 A1
- JP 2247109 A
- US 2008031832 A1
- JP 2007016111 A
- JP HEI3183620 A
- JP HEI11217219 A
- JP 2008266283 A
- JP HEI11302015 A
- JP HEI06127932 A